# EUROPÄISCHE PATENTSCHRIFT

(11) **EP 1 206 294 B1**
(45) Veröffentlichungstag und Bekanntmachung des Hinweises auf die Patenterteilung: **09.11.2005**
(21) Anmeldenummer: 00941863.3
(22) Anmeldetag: 18.07.2000
(51) Int. Cl.: A61M 1/16

(54) **MIKRODIALYSESONDE**
MICRODIALYSIS PROBE
SONDE DE MICRODIALYSE

(30) Priorität: 06.08.1999 DE 19937099
(43) Veröffentlichungstag der Anmeldung: 22.05.2002
(73) Patentinhaber: Disetronic Licensing AG, 3401 Burgdorf (CH)
(72) Erfinder: EHWALD, Rudolf, D-10115 Berlin (DE); BEYER, Uwe, CH-3007, Bern (DE); THOMAS, Andreas, D-01796 Pirna (DE)
(74) Vertreter: Gassenhuber, Andreas
(86) Internationale Anmeldenummer: PCT/CH2000/000389
(87) Internationale Veröffentlichungsnummer: WO 2001/010483

(56) Entgegenhaltungen:
- WO-A-96/14889
- DE-U- 9 002 100
- GB-A- 2 130 916
- US-A- 5 441 481

## Beschreibung

Die vorliegende Erfindung betrifft eine Mikrodialysesonde gemäß dem Oberbegriff des Patentanspruchs 1.

Zur Mikrodialyse werden in Medizin und biologischer Forschung implantierbare Hohlfasern, Hohlfaserschleifen oder Dialysesonden eingesetzt. Die üblichen Dialysesonden besitzen einen röhrenförmigen Schaft, in dem Dialysat abgeleitet wird und der eine geschlossene zylindrische Membran (einseitig verschlossene Hohlfaser) aufweist, in deren Inneres eine dünne Röhre zur Zuführung der Perfusionslösung hineinragt. Zwischen der zurückfließenden Perfusionslösung und dem Umgebungsmilieu kommt es durch Dialyse an der Hohlfasermembran zum Konzentrationsausgleich für permeable Substanzen. Bekannt sind auch Sonden mit dem gleichen prinzipiellen Aufbau, bei denen die Dialysefaser von einem knickfesten Mantel oder Rahmen, aus dem sie teilweise herausragen kann, umgeben ist, wobei sie von diesem gestützt wird. Eine solche Dialysesonde ist beispielsweise aus der DE 33 42 170 C2 bekannt.

Insbesondere bei der Verwendung viskoser Perfusionslösungen oder bei hohen Fließgeschwindigkeiten zeigt sich, daß derartige Dialysesonden nicht optimal durchströmt werden. Bei seitlichem Druck auf die Sonde kann die innere Röhre leicht aus ihrer zentralen Lage gebracht werden und die Strömungsprofile über den Hohlzylinder verändern sich. Die Strömung kann sich an der Seite, wo der Spalt zwischen Außen- und Innenzylinder sehr schmal ist, verlangsamen oder zum Stillstand kommen, während sich an der gegenüberliegenden Seite eine schnell fließende Vorzugsbahn bildet. Im Schaft, in dem das Dialysat abgeführt wird, entsteht außerdem beim Übergang in die Ableitrohre konstruktionsbedingt ein Totraum. Beides führt zur Verzögerung der Gleichgewichtseinstellung.

Zur Lagestabilisierung der inneren Röhre in der Hohlfaser wurde für solche Sonden durch die DE 197 14 087 A1 vorgeschlagen, die innere Kapilare mit einem Profil zu umgeben. Profile mit solch geringem Durchmesser und einer zentralen Bohrung sind jedoch nur mit sehr hohem Aufwand herstellbar.

Die GB 2,130,916 A offenbart eine Dialysesonde mit einer röhrenförmigen Dialysemembrane und Leitungskanälen zur Zufuhr und zum Abfluss von Perfusionsfluid über die Membrane, wobei die Dialysemembrane der Sonde so angeordnet ist, dass sie von einem Gehäuse umgeben wird, welches die Membrane trägt und teilweise freigibt und in welchem die Leitungskanäle angeordnet sind.

Demgemäß ist die Aufgabe der vorliegenden Erfindung, eine Dialysesonde vorzuschlagen, welche die oben genannten Nachteile des Standes der Technik überwindet. Insbesondere soll eine stabile Strömungsführung und damit eine schnelle Gleichgewichtseinstellung garantiert werden. Außerdem soll vermieden werden, daß im Dialysebereich und in den Zu- und Ableitungen strömungshindernde Toträume entstehen.

Diese Aufgabe wird erfindungsgemäß dadurch gelöst, daß sowohl die Zuleitung als auch die Ableitung einer Mikrodialysesonde als getrennte Hohlkanäle jeweils an der Außenwandung der Sonde nebeneinander angeordnet werden.

Die Anordnung für die Zu- und Ableitung ist also erfindungsgemäß nebeneinander, nicht wie beim Stand der Technik ineinander ausgeführt. Damit bilden sowohl die Zuals auch die Ableitung jeweils einen Teil der Außenwand und können durch ihre eigene Struktur oder durch die Bereitstellung von über den entsprechenden Kanälen ausgebildeten Schutzeinrichtungen so stabil ausgestaltet werden, daß mechanische Einflüsse die Strömung der Perfusionslösung nicht behindern. Während sich beim Stand der Technik, beispielsweise gemäß der DE 33 42 170 C2 ein Druck auf den äußeren Hohlzylinder (also die Ableitung) automatisch auf die darin liegende Zuleitung auswirkt, ist dies bei erfindungsgemäß nebeneinander angeordneter Zu- und Ableitung nicht mehr kritisch.

Ein weiterer Vorteil der erfindungsgemäßen Dialysesonde liegt darin, daß die Zu- und Ableitung jeweils einfach gerade am rückwärtigen Teil der Dialysesonde ein- bzw. auslaufen können, und so Strömungsumlenkungen, bei denen sich Toträume ausbilden, weitgehend vermieden werden können.

Bei einer Ausführungsform der erfindungsgemäßen Mikrodialysesonde besteht der in Strömungsrichtung erste Abschnitt der Ableitung aus einer hinter der Umkehrung in die Zuleitung eindringenden Dialysehohlfaser, wobei die Hohlfaser im Bereich der verschlossenen Spitze der Sonde so befestigt ist, daß ein linearer Strömungsverlauf nach der Umkehrung entsteht, während sie an ihrem anderen Ende in einen zweiten stabilen Abschnitt der Ableitung eingedichtet ist. Hierbei wird die Dialysehohlfaser im vollen Querschnitt von der Perfusionslösung in einer Richtung durchströmt und das Dialysat wird linear ohne Richtungsänderung in die Ableitung eingeführt. Die Umkehrung der Strömungsrichtung, wie sie erforderlich ist, um Zuführung und Abführung der Flüssigkeit von einer Seite her zu ermöglichen, erfolgt dabei vor dem Eintritt in die Dialysehohlfaser, so daß die Dialyse selbst nicht durch Störungen in der Strömung behindert wird. Der vorher angesprochen stabile Abschnitt ist bevorzugt eine Röhre, die den äußeren Teil der Ableitung, d.h. deren Stützbauteil ausbildet. Hierbei kann der Teil der Röhre, der im Bereich der Spitze der Sonde über der Hohlfaser liegt, wo die Dialysehohlfaser in die Zuleitung eindringt, einen Stützabschnitt ausbilden, um diesen Teil der Sonde mechanisch zu festigen.

Die Hohlfaser ist vorzugweise austauschbar ausgebildet und auch dementsprechend eingedichtet, wobei die Röhre und insbesondere deren Stützabschnitt Aussparungen aufweist, über welche die Hohlfaser nach außen frei liegt, um die Dialyse durchführen zu können. Hierbei bilden also die Zuleitung und der auf der gegenüberliegenden Seite der Hohlfaser parallel zu diese angebrachte Stützabschnitt einen äußeren Rahmen, der die Hohlfaser gegen die umgebende Gewebematrix mechanisch abschirmt, ohne einen direkten Flüssigkeitskontakt der Hohlfaser mit dem umgebenden Medium zu verhindem. Die Zuleitung und die Ableitung können grundsätzlich getrennte Teile sein, die an der Spitze und der davon abgewandten Seite bei der Montage fest verbunden sind. Besonders günstig ist es jedoch, wenn die Zuleitung und die Ableitung, die in der Spitze eine Strömungsverbindung aufweisen, zu einem Stück integriert sind, beispielsweise über ein Fixierungsmaterial.

Bei einer weiteren bevorzugten Ausführungsform der erfindungsgemäßen Mikrodialysesonde besteht der Strömungskanal für die Perfusionslösung aus einer Hohlfaser mit einem darin eingebrachten Stützprofil, welches die Zuleitung und die Ableitung voneinander abgrenzt, wobei das Stützprofil im Bereich der Strömungsumkehrung Überströmöffnungen aufweist. Auch hier wird also wiederum das Prinzip verwirklicht, daß die Zuleitung und die Ableitung jeweils mit der Hohlfaser einen Teil der Außenwandung der Sonde bilden, jedoch so getrennt und abgestützt sind, daß die Strömung nicht behindert wird. Hierbei wird sowohl die Stützfunktion als auch die Strömungsführung durch das Profil übernommen. Das Stützprofil ist dabei erfindungsgemäß so aufgebaut, daß das durchströmbare Volumen der Hohlfaser aus mehreren langgestreckten Hohlräumen besteht. Diese Hohlräume ermöglichen die Strömung des Perfusionsmittels in die Spitze der Sonde und dessen Rückleitung zur anderen Seite, wobei die Strömungsumkehrung durch die Überströmöffnung erfolgt. Auch hierbei kann die Zu- und Abströmung weitgehend in geradem Strömungsverlauf erfolgen.

Bevorzugt ist eine wie oben beschriebene Ausführung der Mikrodialysesonde so ausgestaltet, daß die Hohlfaser am Zu- und Ableitungsende der Sonde in einen Sondenschaft eingedichtet ist, der die Zuleitung und die Ableitung getrennt aufnimmt und weiterführt. Ein solcher Sondenschaft sorgt für eine weitere Stabilitätserhöhung und ermöglicht es, die erforderlichen Anschlüsse bereit zu stellen.

Das Stützprofil kann im Querschnitt sternförmig ausgebildet sein, beispielsweise als drei- oder vierarmiger Stern. Jedoch ist es andererseits auch möglich, das Profil als flache Scheidewand auszubilden, die einen rechteckigen oder linsenförmigen Querschnitt aufweist und auf einer oder beiden flachen Seiten mit feinen Borsten oder Noppen versehen ist, welche die Hohlfaserwand auf Distanz halten.

Die höchste mechanische Stabilität wird allerdings mit einem Sternprofil erreicht. Bei einem Viersternprofil wird die Perfusionslösung in zwei Kanälen parallel als Zuleitung geführt, während die beiden anderen Kanäle die Ableitung bilden. Bei der Verwendung eines Stemprofils mit drei Armen kann die quellungsbedingte Dehnung des Hohlfasermaterials ausgeglichen werden, wenn die trockene Hohlfaser straff über das Profil geschoben wird und im Querschnitt als Dreieck mit abgerundeten Ecken erscheint. Bei Dehnung der Hohlfasermembran bildet diese dann im Querschnitt wieder einen Kreis. Bei dieser dreiarmigen Ausführung des Profils liegen demnach eine einzige Zuleitung, jedoch zwei Ableitungen vor. Da die Hohlfaser von innen ausreichend gestützt ist, kann sie auf ihrer gesamten Länge zur Gewebematrix hin frei liegen. Dabei erfolgt die Dialyse sowohl in der Zuleitung als auch in den Ableitungen.

Obwohl in der einzelnen Zuleitung aufgrund des geringeren Querschnittes eine höhere Strömungsgeschwindigkeit herrscht, kann auch hier ein effizienter Stoffaustausch stattfinden, da in diesem Bereich der Konzentrationsgradient zwischen Perfusionslösung und Umgebung noch am höchsten ist. Nach dem Überströmen der Perfusionslösung in unmittelbarer Nähe der Spitze in die beiden parallelen Ableitungen halbiert sich die Strömungsgeschwindigkeit, wodurch ein Konzentrationsausgleich für die durch die Hohlfaser hindurch gehenden Substanzen noch gefördert wird, da hierzu mehr Zeit zur Verfügung steht.

Bevorzugt sollten Zuleitung und/oder Ableitung einer erfindungsgemäßen Mikrodialysesonde einen im wesentlichen linearen Verlauf haben, um die Entstehung von Toträumen in der Strömung weitgehend auszuschließen.

Die Erfindung wird im weiteren anhand zweier bevorzugter Ausführungsformen näher erläutert. Es zeigen:
Figur 1 eine erste Ausführungsform einer erfindungsgemäßen Mikrodialysesonde im Längsschnitt mit zwei Querschnittsansichten; und
Figur 2 eine zweite Ausführungsform einer erfindungsgemäßen Mikrodialysesonde im Längsschnitt mit vier Querschnittsansichten.

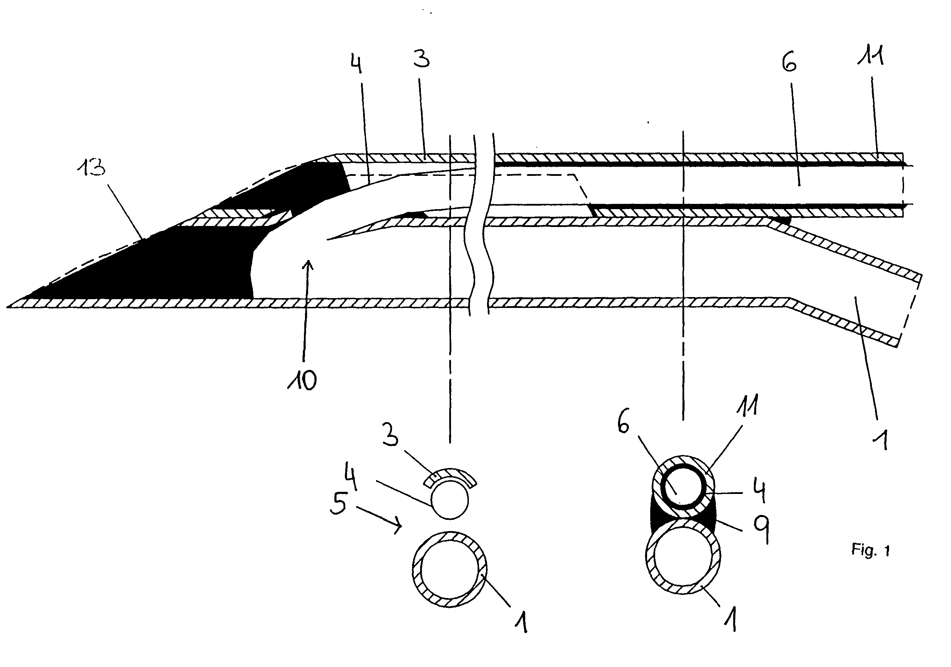

Die in Figur 1 gezeigte Mikrodialysesonde besteht aus einem Zuführungskanal, nämlich einer Zuleitung 1, durch die die Perfusionslösung in die Sonde eingebracht wird. Auf der anderen Seite der Sonde ist diese mit einem Abdichtungsmaterial 13 verschlossen und angespitzt, um das Einführen in das Unterhautgewebe zu ermöglichen. In der Nähe der Spitze der Sonde dringt die Hohlfaser 4 von oben in eine Öffnung 10 der Zuleitung 1 ein. Diese Strömungsverbindung ist mit Hilfe der Formausbildung des Abdichtmaterials so ausgebildet, daß eine die Strömung relativ wenig behindernde Strömungsumkehr stattfinden kann. Andererseits ist die Hohlfaser 4 in der Umgebung der Öffnung 10 mit Hilfe des Abdichtmaterials eingedichtet, so daß keine Lekage entsteht.

Die Hohlfaser 4 bildet nach der Strömungsumkehr den Dialyseabschnitt aus. Zu diesem Zweck ist die Röhre 11, die die Ableitung 6 umgibt, im Bereich des links dargestellten Querschnittes mit Aussparungen 5 versehen, so daß dort die Hohlfaser 4 zum umgebenen Gewebe hin freiliegt und die Dialyse stattfinden kann.

In diesem Bereich liegt lediglich ein Stützabschnitt 3 der Röhre 11 über der Hohlfaser 4, um diese auf dieser Seite gegen mechanischen Druck von außen abzuschirmen.

In ihrem weiteren Verlauf zum rechten Ende der Ableitung 6 hin wird die Hohlfaser 4 dann wieder vollständig von der Röhre 11 umschlossen, wie aus dem rechts dargestellten Querschnitt hervorgeht. In diesem Bereich wird sie mittels eines Trägermaterials (grau dargestellt) in der Röhre 11 eingedichtet. In diesem Bereich sind die Zuleitung 1 und die Ableitung 6 mit der umgebenen Röhre 11 aneinander fixiert und zwar mit dem Fixierungsmaterial 9, das im rechts dargestellten Querschnitt am besten erkennbar wird. Die Dialysesonde bildet so eine integrale Einheit.

Bei der Betrachtung dieser Ausführungsform der erfindungsgemäßen Mikrodialysesonde, wie sie in Figur 1 dargestellt wird, wird nunmehr einerseits deutlich, daß im Strömungsverlauf, insbesondere beim Ein- und Austrag der Perfusionslösung keine Toträume entstehen, welche die Strömung behindern und die Gleichgewichtseinstellung verzögern könnten. Andererseits zeigt sich deutlich, daß diese Nebeneinanderanordnung von Zuleitung 1 und Ableitung 6 es gestattet, eine sehr stabile Sonde auszubilden, bei der auch äußere mechanische Einwirkungen die Kontinuität der Strömung kaum unterbrechen können. Kurz gesagt können, da sowohl die Zuleitung als auch die Ableitung zumindest zum großen Teil an der Außenwand der Sonde parallel angeordnet sind, diese Teile auch mechanisch gut geschützt werden. Dies ist bei in der Ableitung aufgenommenen Zuleitungen, wie sie durch den Stand der Technik bisher bereitgestellt wurden, nicht oder nur mit sehr großem Aufwand möglich.

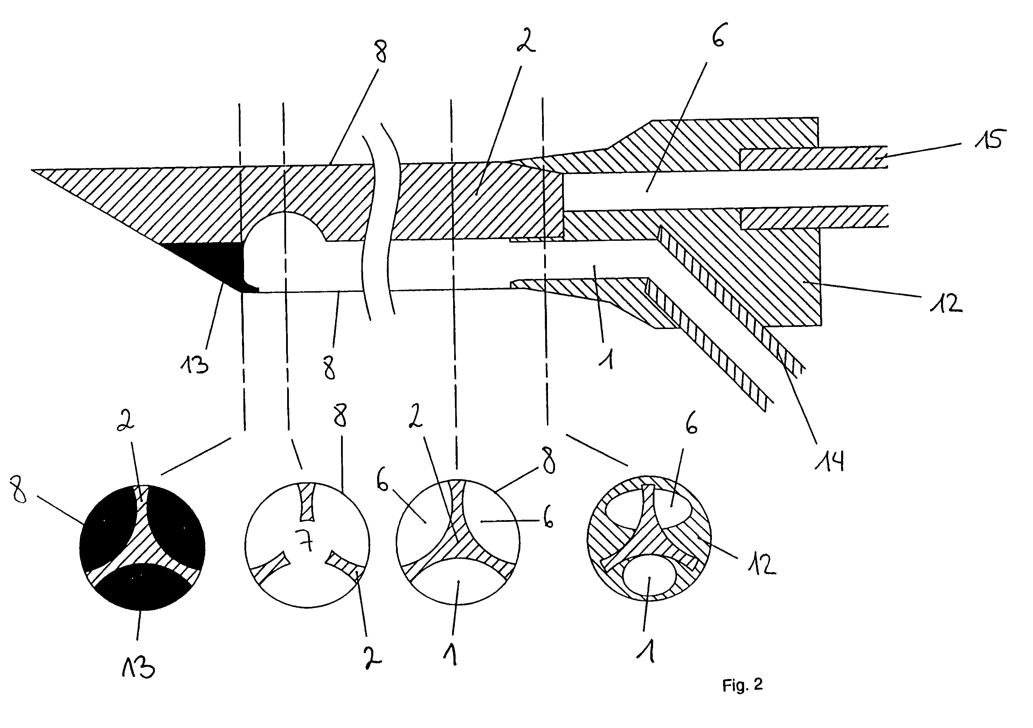

Eine zweite Mikrodialysesonde zeigt in einer anderen Ausführungsform die Figur 2. Die Zu- und Ableitungskanäle 1 und 6 liegen bei dieser Konstruktion am Zu- und Ableitungsende der Sonde in einem Sondenschaft 12, wo eingesetzte Schläuche 14 und 15 angeordnet sind. An der linken Stirnseite des Schaftes 12 ist das dreiarmige sternförmige Profil 2 angesetzt, über welches die Hohlfaser 8 gezogen und am Ansatzpunkt abgedichtet wird. Die Zuleitung 1 und die Ableitung 6 werden im Bereich des Schaftes 12 noch durch diesen selbst ausgebildet, wie aus dem ganz rechts dargestellten Querschnitt hervorgeht. Der zweite Querschnitt von rechts zeigt dann, wie durch das mit der Hohlfaser 8 überzogene Profil 2 eine untere Zuleitung 1 und zwei obere Ableitungen 6 ausgebildet werden. Durch das Profil 2 sind die Zuleitung 1 und die Ableitungen 6 bis nach vorne zum Spitzenbereich hin voneinander getrennt und laufen parallel. Im zweiten Querschnitt von links ist dann dargestellt, daß an dieser Stelle das Mittelstück des Profils 2 ausgespart wurde, so daß eine Überströmöffnung 7 entsteht, durch welche die Perfusionsflüssigkeit aus der Zuleitung 1 in die Ableitungen 6 strömen kann. Hier findet also bei dieser Ausführungsform die Strömungsumkehr statt. An der Spitze ist das Profil zusammen mit der Hohlfaser durch ein Abdichtmaterial 13 verschlossen, wie im linken Querschnitt zu sehen ist. Nach dem Passieren der Überströmöffnung 7 strömt die Perfusionsflüssigkeit dann in den beiden Ableitungen 6 zurück, die sich im Schaftbereich wieder vereinigen. Auch hier laufen die Zuleitung und die Ableitungen nebeneinander und werden von innen her durch das Profil 2 so abgestützt, daß eine Behinderung der Strömung durch äußere Einflüsse weitgehend ausgeschlossen werden kann. Ferner wird auch an diesem Beispiel deutlich, daß die Strömung im wesentlichen linear und über weite Strecken geradlinig geführt wird, so daß Toträume und damit verbundene Strömungsbehinderungen und Verzögerungen der Gleichgewichtseinstellung vermieden werden.

Die Dialyse findet über den gesamten Abschnitt der Hohlfaser 8 vom Schaft 12 bis zum Abdichtungsmaterial 13 hin sowohl in der Zuleitung 1 als auch in den Ableitungen 6 statt. Zwar strömt wegen des geringeren Querschnitts die Lösung in der Zuleitung 1 schneller, jedoch ist in diesem Bereich auch der höchste Konzentrationsgradient vorhanden, so daß eine ausreichende Dialyse stattfindet. In den beiden Zuleitungsabschnitten 6 ist dieser Konzentrationsgradient zwar schon geringer, jedoch ist hier auch die Kontaktfläche größer und die Strömungsgeschwindigkeit beträgt nur die Hälfte, so daß auch in diesem Bereich ein wirksamer Konzentrationsausgleich erzielt werden kann. Die Bauteile 14 und 15 können als Zu- bzw. Ableitungsschläuche ausgestaltet werden und an ihren Einbringstellen in den Schaft 12 in einfacher Weise so abgedichtet sein, daß ein Austreten der Lösung verhindert wird.

## Patentansprüche

1. Mikrodialysesonde, die eine Zuleitung (1) und eine Ableitung (6) für eine Perfusionslösung und einen Dialyseabschnitt umfasst, wobei der Strömungskanal für die Perfusionslösung zwischen Zuleitung (1) und Ableitung (6) im Bereich des Dialyseabschnittes eine Umkehrung erfährt, **dadurch gekennzeichnet, dass** sowohl die Zuleitung (1) als auch die Ableitung (6) als getrennte Hohlkanäle jeweils an der Außenwandung der Sonde nebeneinander angeordnet sind.

2. Mikrodialysesonde nach Anspruch 1, **dadurch gekennzeichnet, dass** der in Strömungsrichtung erste Abschnitt der Ableitung (6) aus einer hinter der Umkehrung in die Zuleitung (1) eindringenden Dialysehohlfaser (4) besteht, während sie an ihrem anderen Ende in einer zweiten stabilen Röhre (11) der Ableitung (6) eingedichtet ist.

3. Mikrodialysesonde nach Anspruch 2, **dadurch gekennzeichnet, dass** der Teil der Röhre (11), der im Bereich der Spitze der Sonde über der Hohlfaser (4) liegt, einen Stützabschnitt (3) ausbildet.

4. Mikrodialysesonde nach Anspruch 2 oder 3, **dadurch gekennzeichnet, dass** die Hohlfaser (4) austauschbar ausgebildet und eingedichtet ist, wobei Röhre (11) und insbesondere der Stützabschnitt (3) Aussparungen (5) aufweist, über welche die Hohlfaser (4) nach außen hin freiliegt.

5. Mikrodialysesonde nach Anspruch 1, **dadurch gekennzeichnet, dass** der Strömungskanal für die Perfusionslösung aus einer Hohlfaser (8) mit einem darin eingebrachten Stützprofil (2) besteht, welches die Zuleitung (2) und die Ableitung (6) voneinander abgrenzt, wobei das Stützprofil (2) im Bereich der Strömungsumkehrung Überströmöffnungen (7) aufweist.

6. Mikrodialysesonde nach Anspruch 5, **dadurch gekennzeichnet, dass** die Hohlfaser (8) am Zu- und Ableitungsende der Sonde in einen Sondenschaft (12) eingedichtet ist, der die Zuleitung (2) und die Ableitung (6) getrennt aufnimmt und weiterführt.

7. Mikrodialysesonde nach einem der Ansprüche 5 oder 6, **dadurch gekennzeichnet, dass** das Profil (2) sternförmig ausgebildet ist, insbesondere als drei- oder vierarmiger Stern.

8. Mikrodialysesonde nach einem der Ansprüche 5 oder 6, **dadurch gekennzeichnet, dass** das Profil flach ausgebildet ist.

9. Mikrodialysesonde nach Anspruch 8, **dadurch gekennzeichnet, dass** das Profil zur Stützung der Hohlfaser auf mindestens einer seiner flachen Seiten Borsten oder Noppen aufweist.

10. Mikrodialysesonde nach einem der Ansprüche 1 bis 9, **dadurch gekennzeichnet, dass** die Zuleitung (1) und/oder die Ableitung (6) einen linearen Verlauf haben.

## Claims

1. A micro-dialysis probe which includes a supply line (1) and a drainage line (6) for a drip-feed solution and a dialysis section, wherein the flow channel for said drip-feed solution experiences an inversion in the area of said dialysis section between said supply line (1) and said drainage line (6), **characterised in that** both said supply line (1) and said drainage line (6) are respectively arranged as separate hollow channels on the outer wall of said probe, side by side.

2. The micro-dialysis probe as set forth in claim 1, **characterised in that** the first section of said drainage line (6) in the direction of the flow consists of a dialysis hollow fibre (4) penetrating into said supply line (1) behind said inversion, while at its other end it is sealed into a second stable tube (11) of the drainage line (6).

3. The micro-dialysis probe as set forth in claim 2, **characterised in that** the part of said tube (11) which lies over said hollow fibre (4) in the area of said tip of said probe forms a supporting section (3).

4. The micro-dialysis probe as set forth in claim 2 or 3, **characterised in that** said hollow fibre (4) is formed to be replaceable and is sealed in, said tube (11) and in particular said supporting section (3) comprising recesses (5) via which said hollow fibre (4) is exposed outwards.

5. The micro-dialysis probe as set forth in claim 1, **characterised in that** the flow channel for said drip-feed solution consists of a hollow fibre (8) with a supporting profile (2) inserted into it, which separates said supply line (1) and said drainage line (6) from each other, said supporting profile (2) comprising overflow openings (7) in the area of flow inversion.

6. The micro-dialysis probe as set forth in claim 5, **characterised in that**, at the supply line end and drainage line end of said probe, said hollow fibre (8) is sealed into a probe shaft (12) which accommodates and continues said supply line (1) and said drainage line (6) separately.

7. The micro-dialysis probe as set forth in any one of claims 5 or 6, **characterised in that** said profile (2) is formed star-shaped, in particular as a three-pointed or four-pointed star.

8. The micro-dialysis probe as set forth in any one of claims 5 or 6, **characterised in that** said profile is formed flat.

9. The micro-dialysis probe as set forth in claim 8, **characterised in that** said profile comprises bristles or knobs on at least one of its flat sides, to support said hollow fibre.

10. The micro-dialysis probe as set forth in any one of claims 1 to 9, **characterised in that** said supply line (1) and/or said drainage line (6) have a linear course.

## Revendications

1. Sonde de microdialyse qui comporte une conduite d'arrivée (1) et une conduite de départ (6) pour une solution de perfusion et une section de dialyse, dans laquelle le canal d'écoulement pour la solution de perfusion entre la conduite d'arrivée (1) et la conduite de départ (6) effectue un demi-tour au niveau de la section de dialyse, **caractérisée en ce que** tant la conduite d'arrivée (1) que la conduite de départ (6) sont juxtaposées respectivement sous la forme de canaux creux séparés sur la paroi extérieure de la sonde.

2. Sonde de microdialyse selon la revendication 1, **caractérisée en ce que** la première section dans la direction d'écoulement de la conduite de départ (6) consiste en une fibre creuse de dialyse (4) pénétrant en amont du retour dans la conduite d'arrivée (1), tandis que, sur son autre extrémité, elle est incorporée dans un second tube stable (11) de la conduite de départ (6).

3. Sonde de microdialyse selon la revendication 2, **caractérisée en ce que** la partie du tube (1) qui se situe dans la zone de la pointe de la zone au-dessus de la fibre creuse (4) forme un tronçon d'appui (3).

4. Sonde de microdialyse selon la revendication 2 ou 3, **caractérisée en ce que** la fibre creuse (4) est conçue et incorporée de façon échangeable, dans laquelle le tube et en particulier le tronçon d'appui creux (3) présentent des évidements (5), par lesquels la fibre creuse (4) repose librement vers l'extérieur.

5. Sonde de microdialyse selon la revendication 1, **caractérisée en ce que** le canal d'écoulement pour la solution de perfusion consiste en une fibre creuse (8) et un profil d'appui (2) incorporé dans celle-ci, qui délimite entre elles la conduite d'arrivée (2) et la conduite de départ (6), dans lequel le profil d'appui (2) présente des ouvertures de trop-plein (7) dans la région de retour d'écoulement.

6. Sonde de microdialyse selon la revendication 5, **caractérisée en ce que** la fibre creuse (8) est insérée de façon étanche au niveau de l'extrémité de la conduite d'arrivée et de départ de la sonde dans un fût de sonde (12) qui reçoit de façon séparée la conduite d'arrivée (2) et la conduite de départ (6) et prolonge leur trajet.

7. Sonde de microdialyse selon l'une des revendications 5 ou 6, **caractérisée en ce que** le profil (2) est conçu en étoile, en particulier en forme d'étoile à trois ou quatre branches.

8. Sonde de microdialyse selon l'une des revendications 5 ou 6, **caractérisée en ce que** le profil est de conception plate.

9. Sonde de microdialyse selon la revendication 8, **caractérisée en ce que** le profil présente pour l'appui de la fibre creuse, au moins sur l'un de ses côtés plats, des poils ou nopes.

10. Sonde de microdialyse selon l'une des revendications 1 à 9, **caractérisée en ce que** la conduite d'arrivée (1) et/ou la conduite de départ (6) ont un tracé linéaire.
